(19) Europäisches Patentamt
European Patent Office
Office européen des brevets

(11) EP 3 406 605 B1

(12) EUROPEAN PATENT SPECIFICATION

(45) Date of publication and mention of the grant of the patent:
**20.04.2022 Bulletin 2022/16**

(21) Application number: **17172281.2**

(22) Date of filing: **22.05.2017**

(51) International Patent Classification (IPC):
***C07D 319/12*** *(2006.01)* ***C08G 63/08*** *(2006.01)*

(52) Cooperative Patent Classification (CPC):
**C07D 319/12; C08G 63/08; C08G 63/78**

(54) **METHODS FOR PRODUCING LACTIDE WITH RECRYSTALLIZATION AND RECYCLE OF MESO-LACTIDE**

VERFAHREN ZUR HERSTELLUNG VON LACTID MIT REKRISTALLISIERUNG UND WIEDERVERWENDUNG VON MESO-LACTID

PROCEDES DE PRODUCTION DE LACTIDE AVEC RECRISTALLISATION ET RECYCLAGE DE MESO-LACTIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**28.11.2018 Bulletin 2018/48**

(73) Proprietor: **NatureWorks LLC**
**Minnetonka, MN 55345 (US)**

(72) Inventors:
- **SCHROEDER, Joseph D.**
  **Minneapolis, MN 55410 (US)**
- **BENSON, Richard Douglas**
  **Long Lake, MN 55346 (US)**

(74) Representative: **Cabinet Beau de Loménie**
**158, rue de l'Université**
**75340 Paris Cedex 07 (FR)**

(56) References cited:
**WO-A1-93/15127**
**WO-A1-2014/180836**
**WO-A2-2010/105143**
**US-A1- 2014 031 566**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 406 605 B1

## Description

This invention relates to methods for making lactide and polylactide (PLA).

Polylactide resins are made industrially by converting lactic acid to lactide, which is then polymerized. Lactic acid is a molecule with one chiral center, and so it exists in two enantiomeric forms, the so-called R- (or D-) enantiomer and the S- (or L-) enantiomer. Two molecules of lactic acid can condense with the elimination of two molecules of water to form a 3,6-dimethyl-1,4-dioxane-2,5-dione, which is commonly referred to as "lactide". Lactide can be considered as being made up of two "lactic units", each of which has the structure:

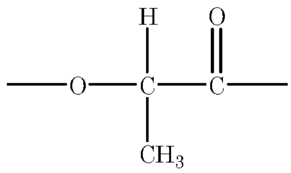

Each lactic unit in a lactide molecule contains one chiral center and exists in either the R- or the S- form. A lactide molecule can take one of three forms: 3S,6S-3,6-dimethyl-1,4-dioxane-2,5-dione (S,S-lactide), 3R,6R-3,6-dimethyl-1,4-dioxane-2,5-dione (R,R-lactide), and 3R,6S-dimethyl-1,4-dioxane-2,5-dione (R,S-lactide or meso-lactide). These have the following structures:

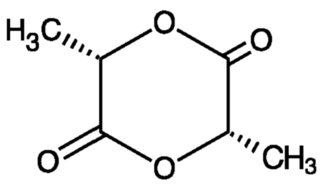

S,S-Lactide

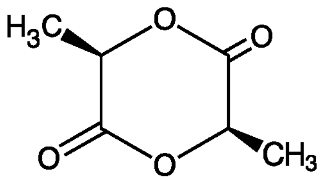

R,R-Lactide

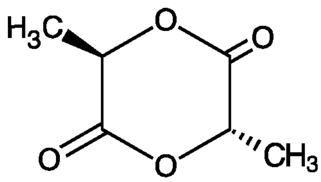

Meso-Lactide

S,S-lactide and R,R-lactide are a pair of enantiomers, while meso-lactide is a stereoisomer.

Polylactide is formed by polymerizing lactide. Polylactide, like lactide, is made up of lactic units. When an S,S-lactide molecule is incorporated into the polylactide polymer chain, it adds two adjacent S-lactic units. R,R-lactide brings in two adjacent R-lactic units, and meso-lactide brings in one S-lactic unit and an adjacent R-lactic unit.

The ratio and distribution of the R- and S-lactic units in the polylactide impact its crystalline behavior and physical properties. When neither the R-lactic units nor the S-lactic units are greatly in excess, the polylactide can crystallize slowly if at all. When one type of lactic unit is more predominant, the polylactide resin is more easily crystallized to form a semi-crystalline polymer. This is usually the case when the ratio of the two enantiomeric forms exceeds about 90:10 or especially exceeds 92:8. As the enantiomer ratio increases from about 90:10 towards 100:0, the polylactide tends to crystallize more easily and becomes capable of developing a greater amount of crystallinity. It is not important whether it is the R- or S- form that is predominant. However, S-lactic units usually predominate because most industrial processes produce lactic acid in fermentation processes and most microorganisms that are used in fermentation processes produce the S-enantiomer.

Processes that are suitable for large-scale production of polymer grade lactide from lactic acid are described, for example, in U. S. Patent Nos. 5,247,058, 5,258,488, 5,357,035, 5,338,822, 6,005,067, 6,277,951, 6,326,458 and WO 93/15127. The processes described in these patents generally involve forming a low molecular weight poly(lactic acid), and then depolymerizing the low molecular weight poly(lactic acid). The depolymerization step produces lactide. The lactide is then purified to separate it from water, residual lactic acid, linear lactic acid oligomers and other impurities as may be present. This can be done by distillation or by other methods such as recrystallization, either from a solvent or from a melt.

The starting lactic acid is usually of very high optical purity. However, the starting material is subjected to elevated temperatures as it is converted to the low molecular weight poly(lactic acid) polymer and subsequently depolymerized. Some racemization (*i.e.*, conversion of one enantiomeric form to the other) occurs under those conditions. In addition, a much smaller amount of racemization may occur as the lactide is purified. Perhaps 1 to 10% of the lactic units in the starting material may become racemized in this fashion to form the other enantiomer, although this amount can vary quite substantially in any given manufacturing process. Because this racemization occurs, the lactide obtained from the process will be a mixture of S,S-lactide, R,R-lactide and meso-lactide.

It is sometimes desirable to separate some or all of the meso-lactide from the S,S-lactide and R,R-lactide. There is also a need to remove various types of impurities that form as lactic acid is converted to lactide and the lactide is purified.

One way to separate meso-lactide and impurities from S,S-lactide and R,R-lactide is by distillation. Unfortunately, some of the most prevalent impurities happen to have volatilities that lie between those of S,S- and R,R-lactide, on the one hand, and meso-lactide on the other. Some of these have volatilities very close to that of meso-lactide. These "intermediate-boiling" impurities can be characterized by their relative volatility in a lactide matrix at a given set of distillation conditions; they tend to have relative volatilities of 1.001 to 1.5 relative to S,S-lactide when distilled from a lactide matrix, and typically have boiling points in the range of about 155 to about 180°C at 50 Torr pressure. These intermediate-boiling impurities and the meso-lactide distill off together and form a meso-lactide stream in which those impurities are concentrated. This is an effective way to produce a purified S,S-lactide and R,R-lactide stream, but comes at the cost of producing a meso-lactide stream laden with the impurities.

The exact structure and identity of these "intermediate-boiling" impurities is not entirely known, although they are believed to include compounds such as succinic acid and 2-methylsuccinic acid. These intermediate-boiling impurities or reaction products that form from them when the lactide is polymerized impart color to the polymer; thus the color (typically expressed as "yellowness" of the polymer) is an indicator of the extent these impurities were present in the lactide stream.

US 2014/0031566 describes a process for recovering lactic acid values from a starting lactide composition, wherein said composition is subjected to a temperature of up to 170°C in the presence of a racemization catalyst. WO 2014/180836 describes a process for the recovery and production of meso-lactide from a crude lactide-containing stream, comprising distillation and melt crystallization steps.

To improve process economics, it is desirable to recover and re-use the meso-lactide. This is quite problematic in practice, because it is difficult to separate the meso-lactide from the intermediate-boiling impurities and if the intermediate-boiling impurities are not removed they will cause yellowness when the meso-lactide is polymerized. In actual practice, the difficulty and cost of removing the impurities from the meso-lactide has been such that the meso-lactide is usually discarded or used in other, lower-value applications. This reduces overall yields and increases the overall cost of the process.

Therefore, it would be desirable to reduce these yield losses and provide a more efficient method for producing a polymerizable lactide stream. It would also be desirable to provide an effective method of producing a highly purified lactide stream which is useful for preparing semi-crystalline grades of polylactide.

WO 2010/105143 describes an approach to resolving these problems. In the process described in WO 2010/105143, the impurity-laden meso-lactide stream is purified using one or more of several approaches, and the purified meso-lactide is recycled to an early point in the process. The recycled meso-lactide is re-incorporated into prepolymer where it loses its identity, and the prepolymer is then depolymerized to form new lactide molecules. This process allows meso-lactide to be reprocessed, but it changes the ratios of R,R-, S,S- and meso-lactide. In the usual case, the amount of the less predominant enantiomer (the R,R-lactide in most industrial processes) tends to build up in the system. As described in WO 2010/105143, these changing ratios can be tolerated surprisingly well, but eventually the enantiomer distribution can become such that it is difficult to prepare semi-crystalline grades of polymer. The amount of meso-lactide that can be recycled in this way is limited.

This invention is a process for producing a polylactide, comprising:

a) forming a low molecular weight poly(lactic acid);
b) depolymerizing the low molecular weight poly(lactic acid) to form a crude lactide that includes meso-lactide, S,S-lactide and R,R-lactide, wherein either S,S-lactide or R,R-lactide is the non-predominant lactide, and the crude lactide further includes intermediate-boiling impurities;
c) separating meso-lactide from the crude lactide in one or more steps such that

1) a meso-lactide-enriched stream is formed in which the mole fraction of meso-lactide is at least 0.8 and which in which the intermediate-boiling impurities are concentrated; and
2) a purified S,S- and R,R-lactide stream is formed;

d) purifying the meso-lactide-enriched stream by melt crystallization to separate meso-lactide from intermediate-boiling impurities and form a purified meso-lactide stream;
e) recombining at least a portion of the purified meso-lactide stream with at least a portion of the purified S,S- and R,R-lactide stream to form a recombined stream containing 4 to 25% by weight of the purified meso-lactide stream based on the combined weight of the purified meso-lactide stream and the purified S,S- and R,R-lactide stream, and polymerizing the recombined stream.

Because a lower level of these impurities can be obtained, one can reduce or even eliminate the capital and/or operating costs associated with removing those impurities from the S,S- and R,R-lactide stream before it is polymerized, or from the polymer after it is polymerized.

The FIGURE is a schematic diagram of an embodiment of a process according to the invention.

The embodiment illustrated in The Figure illustrates various preferred or optional features. The Figure is not intended to show specific engineering features or details, including the design of the various components shown. In addition, auxiliary equipment such as various valves, pumps, heating and cooling equipment, analytical, control devices and the like are not shown, but of course can be used as necessary or desirable.

The general process for producing lactide by forming a low molecular weight poly(lactic acid), followed by depolymerizing the low molecular weight poly(lactic acid) to form lactide is well-known and described, for example, in 5,247,058, 5,258,488, 5,536,807, 5,357,035, 5,338,822, 6,005,067, 6,277,951, 6,310,218 and 6,326,458 and WO 95/09879. Except for the recycling of the meso-lactide stream as described herein, the processes described in those and similar references can be conducted with regard to this invention in the general manner described in those references, and those process steps will be described only briefly below.

The low molecular weight poly(lactic acid) is a polymer of lactic acid units, suitably prepared by forming a concentrated lactic acid or lactic acid derivative stream that contains 60 to 95% by weight lactic acid or lactic acid derivative in water or, less preferably, another solvent. The lactic acid derivative may be, for example, a lactic acid ester, a lactic acid salt, a lactic acid oligomer, and the like. The starting lactic acid or lactic acid derivative should contain at least 90%, preferably at least 95%, more preferably at least 98% and still more preferably at least 99% of either the S- or R-enantiomer, and no more than 10%, preferably no more than 5%, more preferably no more than 2% and even more preferably no more than 1% of the other enantiomer. This stream may contain some oligomeric species that form as the lactic acid or derivative is concentrated. The stream is then further concentrated by removing water (or a lower alcohol in the case of a lactic acid ester) and solvent (if any) in an evaporator. This causes the lactic acid or lactic acid derivative to condense, eliminating water or a lower alcohol as the condensation by-product. As this is an equilibrium reaction, the removal of condensation products favors the further condensation of the lactic acid or derivative. A low molecular weight poly(lactic acid) formed this way has a number average molecular weight of up to about 5000, preferably from 400 to 3000. The low molecular weight poly(lactic acid) may also include non-lactic chemical species that are sometimes added to control molecular weight or improve specific processing properties.

The low molecular weight poly(lactic acid) is then depolymerized by subjecting it to an elevated temperature and subatmospheric pressures, typically in the presence of a depolymerization catalyst. Conditions are generally selected to (1) minimize residence time, as doing so reduces the amount of racemization that can occur, and (2) vaporize the crude lactide that is formed. Like the polymerization reaction, the depolymerization is an equilibrium reaction. Removal of the lactide as it is formed favors the production of additional lactide. Therefore, continuous removal of crude lactide is preferred. One or more stabilizers can be present during this step as described in WO 95/09879.

The crude lactide formed in the depolymerization step contains a mixture of S,S-lactide, meso-lactide and R,R-lactide. It often contains various types of impurities, such as residual water, some lactic acid (or alcohol and ester, if esters are used as the starting material), some linear oligomers of lactic acid, and usually some other reaction by-products, which include some intermediate-boiling impurities as described before. Meso-lactide is separated from the crude lactide. This can be done by methods such as melt crystallization, but a preferred method is to fractionally distill the crude lactide stream in one or more steps.

The crude lactide may undergo one or more purification steps prior to or simultaneously with this separation. For example, the lactide may be partially condensed to separate it from more volatile impurities. Alternatively, the crude lactide can be purified by melt crystallization methods as described in U. S. Patent No. 6,310,218, or by solvent crystallization methods. A third approach is to distill off some or all of the impurities that are significantly more volatile than meso-lactide, such as water, residual lactic acid or lactic ester starting materials, and other small organic compounds. Such a distillation step can be performed prior to or simultaneously with a fractional distillation step(s) in which meso-lactide is separated from S,S- and R,R-lactide. It is generally preferred to remove most of the more volatile impurities from the crude lactide before separating out meso-lactide.

The separation step or steps are conducted under conditions sufficient to produce at least one meso-lactide enriched stream and at least one purified S,Sand R,R-lactide stream. The purified S,S- and R,R-lactide stream contains the bulk of the S,S- and R,R-lactide that were present in the crude lactide. If a distillation approach is used, other process streams may be taken from the distillation step or steps. These may include a lights stream (if those materials are not removed beforehand) and a bottoms stream that contains materials that are less volatile than S,S- or R,R-lactide, as well as one or more additional impurity streams.

The process may be operated such that the meso-lactide is not completely separated out, in which case the purified S,S- and R,R-lactide stream will contain some meso-lactide. However, the purified S,S- and R,R-lactide stream is in each case depleted in meso-lactide relative to the meso-lactide stream, meaning that the mole fraction of meso-lactide in the purified S,S- and R,R-lactide stream is lower than in the meso-lactide stream and than in the crude lactide.

In addition, the S,S- and R,R-lactide stream is depleted of impurities, preferably including "intermediate-boiling" impurities as described before. "Depletion" in this case is with reference to the crude lactide stream immediately prior to the separation of the meso-lactide; the weight ratio of these impurities to the lactide content of the S,S- and R,R-lactide stream is lower than the weight ratio of the impurities to the lactide content of the crude lactide stream. This relationship can be expressed by the inequality

$$1 > \frac{I_{SR}/(I_{SR}+L_{SR})}{I_{crude}/(I_{crude}+L_{crude})} \quad \text{(Equation 1)}$$

where $I_{SR}$ represents the weight of the impurities in the S,S- and R,R-lactide stream, $L_{SR}$ represents the weight of lactide in the S,S- and R,R- lactide stream, $I_{crude}$ represents the weight of the impurities in the crude lactide stream immediately prior to the separation of the meso-lactide and $L_{crude}$ represents the weight of lactide in the crude lactide stream immediately prior to the separation of the meso-lactide. Preferably, the ratio in equation 1 is less than 0.1, more preferably less than 0.05 and even more preferably less than 0.01. An advantage of the invention is that an S,S- and R,R-lactide stream can be produced that has very low levels of the impurities.

The impurity level in the purified S,S- and R,R-lactide stream is affected by how completely the meso-lactide is separated from the S,S- and R,R-lactide stream. Many impurities tend to partition disproportionately towards the meso-lactide stream, although some amount typically will remain with the purified S,S- and R,R-lactide stream. The more completely the meso-lactide is separated from the purified S,S- and R,R-lactide stream, the lower the content of impurities in the purified S,S- and R,R-lactide stream will be. Accordingly, it is preferred to reduce the level of meso-lactide in the purified S,S- and R,R-lactide stream as much as possible, consistent with the necessary enantiomeric composition of the stream that goes forward to be polymerized, to reduce the level of impurities in that stream.

Preferably, the separation is conducted under conditions which produce a purified S,S- and R,R-lactide stream that contains a mole fraction of 0.05 or less of meso-lactide (i.e., no more than about 5 mole-% meso-lactide based on the total number of moles of lactide in the stream). The S,S- and R,R-lactide stream more preferably contains a mole fraction of 0.02 or less, even more preferably of 0.01 or less, of meso-lactide. The S,S- and R,R-lactide stream may, for example, contain a mole fraction of from 0 to 0.01, from 0 to 0.005 or from 0 to 0.003 of meso-lactide.

All mole fractions described herein are based on the total moles of lactide in the stream under discussion.

The meso-lactide-enriched stream contains mainly meso-lactide. When step c) is performed in a distillation column, the meso-lactide-enriched steam is taken from the column as a gas. The meso-lactide-enriched stream typically contains at least 60% by weight of meso-lactide, and may contain at least 80% or at least 90% by weight of meso-lactide, based on the total weight of the stream. It may contain small quantities of S,S- or R,R-lactide, but these together generally constitute no more than about 15%, preferably no more than 10% and even more preferably no more than 5% by weight of the lactide content of the stream. The mole fraction of the meso-lactide (relative to all lactide species) in this stream should be at least 0.80. Thus, the meso-lactide-enriched stream is enriched in meso-lactide, compared with the S,S- and R,R-lactide stream and compared with the crude lactide stream.

Intermediate-boiling and other impurities tend to become concentrated in the meso-lactide-enriched stream when the meso-lactide is separated from the crude lactide stream, and so the meso-lactide-enriched stream becomes enriched in those impurities, relative to the crude lactide stream just prior to the separation, as expressed by the inequality

$$1 < \frac{I_{meso}/(I_{meso}+L_{meso})}{I_{crude}/(I_{crude}+L_{crude})} \quad \text{(Equation 2)}$$

where Imeso represents the weight of the impurities in the meso-lactide-enriched stream, $L_{meso}$ represents the weight of lactide in the meso-lactide-enriched stream, and $I_{crude}$ and $L_{crude}$ are as defined with respect to equation 1.

In the Figure, lactic acid or lactic acid derivative stream 5 containing water or, less preferably, another solvent, is fed into prepolymer reactor 1. The lactic acid or lactic acid derivative concentration in a feed stream such as stream 5 preferably is at least 60% by weight, and may be as high as 95% by weight, preferably as high as 90% by weight. Lactic acid may be obtained from a fermentation broth, which is preferably concentrated to within the aforementioned ranges in an upstream process step, which is not shown in the Figure. The starting material is heated in prepolymer reactor 1 to cause the lactic acid or lactic acid ester to condense to form a low molecular weight poly(lactic acid) as described before. Most of the water and solvent (if any) are removed from prepolymer reactor 1 as stream 7. In addition, the condensation by-products formed in the polymerization reaction (water in the case of lactic acid, a lower alcohol in the case of a lactic acid ester) are also mainly removed as part of stream 7. Stream 7 can be discarded, or all or any

portion of it can be recycled to an earlier stage in the process or into an upstream fermentation process for producing lactic acid or lactic acid derivative. Any recycled portion of stream 7 can be purified before being recycled, and if desired part of stream 7 can be taken as a purge stream to remove impurities from the process.

Prepolymer reactor 1 is essentially a reactor and evaporator, which can be of any convenient design. As with all other process steps in which the lactic acid and its derivatives are exposed to elevated temperatures, racemization can occur in prepolymer reactor 1. Accordingly, the prepolymer reactor and other equipment are preferably designed to provide short contact times and to minimize process temperatures, such as through the use of subatmospheric pressures to reduce needed operating temperatures.

Low molecular weight poly(lactic acid) stream 6 is removed from prepolymer reactor 1 and transferred to lactide reactor 2, where it is depolymerized to form lactide. Lactide reactor 2 is again essentially an evaporator, and can be of many types as described in WO95/09879. Examples of suitable lactide reactors include, for example, forced circulation, short path or short tube, long-tube vertical, long-tube horizontal, falling film, agitated thin-film and disk evaporators. Film-generating evaporators, especially falling film and agitated falling film evaporators as described in WO 95/09879, are especially preferred. Various types of staged reactors are also suitable. Lactide reactor 2 is preferably operated at a pressure of from about 1 to about 100 mm Hg, more preferably from about 2 to about 60 mm Hg. An elevated temperature, preferably from about 180 to 300°C and more preferably from 180 to 250°C, is used.

The depolymerization reaction that occurs in lactide reactor 2 is usually catalyzed. In the embodiment shown, catalyst is introduced to prepolymer stream 6 upstream of lactide reactor 2, through catalyst stream 18. It is also possible to introduce catalyst stream 18 directly into lactide reactor 2.

Crude lactide and a bottoms mixture are formed in lactide reactor 2. The bottoms mainly include high boiling materials and higher oligomers of lactic acid. The bottoms are withdrawn as bottoms stream 9. These can be discarded or recycled, with or without treatment, to an earlier step in the process.

The crude lactide produced in lactide reactor 2 contains mainly meso-, S,S- and R,R-lactide, water, lactic acid, and some linear lactic acid oligomers mainly having a degree of polymerization of up to about 6. Other impurities, including intermediate-boiling impurities, may be present. The lactide concentration in the crude lactide is generally in excess of 80% by weight.

Crude lactide formed in lactide reactor 2 is withdrawn as stream 8 and, in the embodiment shown, is transferred to distillation column 3. Crude lactide stream 8 usually is in the form of a vapor. In the embodiment shown, the crude lactide is distilled in three stages, in first distillation column 3, second distillation 4 and third distillation column 20, respectively. It is possible in principle at least to carry out this distillation in a single column or only two distillation columns, or to carry it out in a greater number of columns. The three-stage distillation process illustrated in The Figure has the advantages of simplifying the equipment that is needed in each stage, raising overall processing rates, and allowing conditions in each stage to be optimized for one or more specific separations that are to take place in that particular stage. It is particularly preferred to remove the more volatile impurities such as water and lactic acid from the lactide stream before separating the meso-lactide from the S,S- and R,R-lactide, especially when the meso-lactide is removed by fractional distillation as shown in the Figure.

Crude lactide stream 8 may be partially or fully condensed, if desired, before being sent onward to the separation step. In the embodiment shown, crude lactide stream 8 is introduced into first distillation column 3, where it is separated into partially purified crude lactide stream 10 and overhead stream 12. A bottoms stream (not illustrated) also may be withdrawn from first distillation column 3. Overhead stream 12 contains most of the water and lactic acid that were contained in crude lactide stream 8, together with a small portion of the lactide. The lactic acid portion of overhead stream 12 may include lactic acid that is carried over from lactide reactor 2, and may also include some lactic acid that is regenerated in first distillation column 3. Overhead stream 12 also contains other impurities and reaction by-products that are more volatile than meso-lactide, and may contain some amount of intermediate-boiling impurities. Overhead stream 12 may be discarded, but the lactic acid values at least are preferably recycled into an earlier step of the process, preferably directly or indirectly into step a) (as shown) or into the lactide reactor.

Partially purified crude lactide stream 10 contains S,S-lactide, R,R-lactide, meso-lactide, most of the intermediate-boiling impurities, and some high-boiling impurities such as linear lactic acid oligomers. It is normally substantially devoid of water and lower-boiling impurities.

In the embodiment shown, partially purified crude lactide stream 10 is transferred to second distillation column 4, where lactide is separated from higher-boiling impurities such as linear lactic acid oligomers. This produces purified lactide stream 21 and a bottoms stream 11. Purified lactide stream 21 contains intermediate-boiling impurities as described before, and may still contain some more volatile impurities. Some volatiles (mainly water and lactic acid) may in addition be removed from second distillation column 4 via line 22. Bottoms stream 11 mainly includes high boiling materials and higher oligomers of lactic acid in a lactide matrix. It may be discarded or recycled to an earlier stage in the process; in the embodiment shown, bottoms stream 11 and volatiles removed via line 22 are both recycled via line 19 into lactide reactor 2 to recover lactic acid values from those streams.

Purified lactide stream 21 is then separated to remove meso-lactide and form a purified S,S- and R,R-lactide

stream. In the embodiment shown in the Figure, purified crude lactide stream 21 is transferred to third distillation column 20, where meso-lactide is separated from S,S- and R,R-lactide. As shown, this produces a purified S,S-lactide/R,R-lactide stream 13, which is withdrawn from near or at the bottom of third distillation column 20; a meso-lactide-enriched stream 14, which is withdrawn from near or at the top of third distillation column 20, and an optional intermediate stream 15, which contains intermediate-boiling impurities and meso-lactide. In a commercial scale operation, it may be difficult to withdraw a significant intermediate stream 15 without also withdrawing a large amount of meso-lactide, and so taking such an intermediate stream may not be practical and can be omitted. A bottoms stream (not shown) may also be removed from third distillation column 20.

Impurities that are introduced into third distillation column 20 with purified crude lactide stream 21 will partition between meso-lactide-enriched stream 14 and purified S,S-and R,R-lactide stream 13 (as well as intermediate stream 15 if such a stream is withdrawn). The highly volatile and intermediate-boiling impurities will become more concentrated in meso-lactide-enriched stream 14 and intermediate stream 15 (if taken).

The meso-lactide-enriched streams 14 and/or 15 (if taken) are purified by melt crystallization. The meso-lactide-enriched streams are condensed and brought to a temperature above the melting temperature of meso-lactide. In the embodiment diagrammed in the Figure, meso-lactide-enriched stream 14 is taken to optional condensate collection tank 17 where distillate is gathered. The condensed meso-lactide-enriched stream is then transferred via line 14A to crystallizer 30.

Alternatively, the condensation of meso-lactide stream 14 can occur within crystallizer 30, but this is less preferred because crystallizers 30 typically operate in batch mode, and it would be necessary to include duplicate crystallizers 30 so that one can be processing material as the other is being loaded with distillate.

The condensed meso-lactide stream (14A in the Figure) is cooled in crystallizer 30 to form meso-lactide crystals. Various types of melt crystallization equipment is suitable as crystallizer 30 for purifying condensed meso-lactide stream 14A, including, for example, static melt crystallizers, falling film melt crystallizers, suspension crystallizers, suspension mixed product removal crystallizers, and the like. Suitable melt crystallization equipment is available commercially from, for example, GEA Messo PT (NL) and Sulzer Chemtech (CH).

Intermediate-boiling impurities as well as any other impurities are largely excluded from the meso-lactide crystals that form in crystallizer 30. The exclusion of impurities produces a purified meso-lactide. The resulting crystals are then separated from the remaining liquid (the mother liquor), either within crystallizer 30 or in separate equipment (not shown). The separated crystals may be further purified by, for example, washing, "sweating" (by heating them close to their melting temperature to exclude entrapped impurities), recrystallization or other suitable methods, either within crystallizer 30 or in auxiliary equipment (not shown). The mother liquor and other waste streams that are highly concentrated in the intermediate-boiling impurities can then be discarded or otherwise removed from the process as through line 36 in the Figure.

The purified meso-lactide stream may contain, for example, up to 250 ppm acetic acid; up to 400 ppm succinic acid, up to 75 ppm pyruvic acid, up to 125 ppm levulinic acid, up to 1000 ppm 2,3-butanediol and/or up to 750 ppm 2-methyl succinic acid. The amounts of one or more of acetic acid, succinic acid, pyruvic acid, levulinic acid, 2,3-butanediol and 2-methyl succinic acid may be reduced by at least 50%, at least 60% or at least 70% in the melt crystallization step.

At least a portion of the purified meso-lactide is recombined with the purified S,S- and R,R-lactide stream and the recombined streams are polymerized. For ease of handling, it is generally convenient to re-melt the meso-lactide crystals prior to the recombination and/or polymerization step. In the embodiment shown, purified meso-lactide stream 33 is withdrawn from crystallizer 30 and stored in optional tank 31. At least a portion of the purified meso-lactide is transferred via line 34 to polymerization unit 23 for polymerization. It is noted that the purified meso-lactide crystals should be melted or vaporized prior to transfer to polymerization unit 23 or within polymerization unit 23. This can be performed, for example, in crystallizer 30, in tank 31, in lines 33 or 34, or in various types of auxiliary equipment not shown.

In the embodiment shown, the purified meso-lactide stream 34 is combined with purified S,S- and R,R-lactide stream 13 (which is taken from third distillation column 20) and fed into polymerization unit 23. Alternatively, purified meso-lactide stream 34 and purified S,S- and R,R-lactide stream 13 can be fed separately into polymerization unit 23 and recombined there. This recombined lactide streams are polymerized in polymerization unit 23 to produce polylactide stream 37. The polymerized product preferably is devolatilized to produce devolatilization stream 24. As shown, stream 24 is recycled into lactide reactor 2 via line 19, although stream 24 can instead be discarded or recycled elsewhere into the process.

The relative amounts of the purified meso-lactide stream and purified S,Sand R,R-lactide streams fed into the polymerization unit are selected in known manner to produce the desired lactic acid enantiomer ratio in the product independent of the co-monomer content, the sum of meso-lactide and minor isomer of either R,R-lactide or S,S-lactide, while producing a lactide feed that is low in "intermediate boiling" impurities. The purified meso-lactide stream constitutes at least 4% of the combined weight of the purified meso-lactide stream and the purified S,S- and R,R-lactide stream, up to 25%, such as up to 10%, up to 8% or up to 6% on the same basis.

No special polymerization methods are required. A particularly suitable process for preparing the polylactide

resin is described in U. S. Patent Nos. 5,247,059, 5,258,488 and 5,274,073. In the process described in those patents, lactide is fed as a liquid directly to a polymerization system, where it is polymerized at elevated temperature in the presence of a catalyst. As molecular weight increases, an equilibrium is established between the polymer and free lactide, thus limiting the build-up of molecular weight and producing a polymer containing a certain amount of free lactide. The free lactide provides some plasticizing effect that is often undesirable and also tends to coat the surfaces of polymer processing equipment. For these reasons, the polymerization process typically includes a devolatilization step during which the free lactide content of the polymer is reduced, preferably to less than 1% by weight, and more preferably less than 0.3% by weight.

The polymerization can be conducted batch-wise, semi-continuously or continuously. Continuous stirred tank reactors (CSTRs) and tube or pipe reactors are suitable types of polymerization vessels. A series of CSTRs or tube or pipe reactors may be used to conduct the polymerization in stages. This permits additives to be introduced at specific stages in the polymerization process if desired, and also allows for different reaction conditions to be used at different stages of the polymerization.

Suitable polymerization temperatures preferably are (for solventless processes) above the melting temperature of the monomer or monomer mixture and above the melting temperature of the product copolymer, but below the temperature at which significant polymer degradation occurs. A preferred temperature range is from about 100°C to about 220°C. A more preferred temperature range is from 120°C to about 200°C and especially from about 160°C to about 200°C. Residence times at polymerization temperatures are selected to produce a polymer of the desired molecular weight and/or desired conversion of monomers. Molecular weight control agents such as described in USP 6,277,951 can also be added to obtain the desired molecular weight.

Molecular weight and conversion are controlled by polymerization time and temperature, the equilibrium between free lactide and the polymer, and by the use of initiator compounds. In general, increasing quantities of initiator compounds on a molar basis will tend to decrease the molecular weight of the product polymer.

The polymerization is conducted in the presence of a metal-containing catalyst. Examples of these catalysts include various tin compounds such as $SnCl_2$, $SnBr_2$, $SnCl_4$, $SnBr_4$, SnO, tin (II) bis(2-ethyl hexanoate), butyltin tris(2-ethyl hexanoate), hydrated monobutyltin oxide, dibutyltin dilaurate, tetraphenyltin and the like; PbO, zinc alkoxides, zinc stearate, compounds such as aluminum alkoxides, compounds such as antimony triacetate and antimony (2-ethyl hexanoate), compounds such as bismuth (2-ethyl hexanoate), calcium stearate, magnesium stearate, certain yttrium and rare earth compounds such as are described in U. S. Patent No. 5,208,667 to McLain et al., and the like. Catalysts are used in catalytically effective amounts, which depend somewhat on the particular catalyst, but are usually in the range of 1 mole of catalyst per 3000 to 50,000 moles of monomers. Preferred catalyst concentrations are not more than one mole of catalyst per 5000 moles of monomers, and especially not more than one mole of catalyst per 10,000 moles of monomers.

The resulting PLA resin contains metal catalyst residues, which are preferably deactivated by contacting the PLA resin with a deactivating agent.

If desired, a portion of the purified meso-lactide can be recycled into steps a) and/or b) of the process, as described in 2010/105143. For example, a portion of the purified meso-lactide can be recycled via stream 35 into any or all of feed stream 5 (via line 35A), prepolymer reactor 1 (via line 35B) and lactide reactor 2 (via line 35C), all of which are optional.

## Example

Lactic acid is fed into prepolymer reactor 1 to form a low molecular weight prepolymer that is depolymerized in lactide reactor 2 to form a crude lactide. The crude lactide is distilled in three stages corresponding to distillation columns 3, 4 and 20 to form meso-lactide stream 14 and S,S- and R,R-lactide stream 13.

Samples from streams 13 and 14 are separately polymerized by mixing them with tin bis(2-ethylhexanoate) at a mole ratio of 30,000:1 lactide:tin, and heating to 180°C. Conversion to polymer is monitored in each case. When conversion reaches 90%, samples are withdrawn and the yellowness index of the polymer is measured according to ASTM D-1925 [C/2].

The yellowness index from polymerized stream 13 is 58.4 and that from polymerized stream 14 is higher at 79.6. This is consistent with more of the intermediate-boiling impurities being concentrated in the meso-lactide stream 14.

Stream 14 contains 441 ppm acetic acid, 934 ppm succinic acid, 125 ppm pyruvic acid, 230 ppm levulinic acid, 4280 ppm 2,3-butanediol and 1644 ppm 2-methyl succinic acid. A portion of stream 14 is processed through a melt crystallizer to produce stream 34. This reduces the acetic acid content to 138 ppm, succinic acid content to 268 ppm, pyruvic acid content to 125 ppm, levulinic acid content to 71 ppm, 2,3-butanediol content to 430 ppm and the 2-methyl succinic acid concentration to 445 ppm. When a portion of stream 34 is polymerized by itself, the resulting polymer has a yellowness index of only 40.0.

A mixture of 95% stream 13 and 5% stream 34 is polymerized, and has a yellowness index of 52.5. This is less

than the yellowness index obtained when stream 13 is polymerized by itself, and demonstrates the effectiveness of removing impurities from the system by melt crystallizing the meso-lactide stream. When a mixture of 95% stream 13 and 5% stream 14 is polymerized, the resulting polymer has a yellowness index of 58.1, which is essentially unchanged from the yellowness index obtained when stream 13 is polymerized by itself.

## Claims

**1.** A process for producing a polylactide, comprising:

a) forming a low molecular weight poly(lactic acid);
b) depolymerizing the low molecular weight poly(lactic acid) to form a crude lactide that includes meso-lactide, S,S-lactide and R,R-lactide, wherein either S,S-lactide or R,R-lactide is the non-predominant lactide, and the crude lactide further includes intermediate-boiling impurities;
c) separating meso-lactide from the crude lactide in one or more steps such that

1) a meso-lactide-enriched stream is formed in which the mole fraction of meso-lactide is at least 0.8 and which in which the intermediate-boiling impurities are concentrated; and
2) a purified S,S- and R,R-lactide stream is formed;

d) purifying the meso-lactide-enriched stream by melt crystallization to separate meso-lactide from intermediate-boiling impurities and form a purified meso-lactide stream;
e) recombining at least a portion of the purified meso-lactide stream with at least a portion of the purified S,S- and R,R-lactide stream to form a recombined stream containing 4 to 25% by weight of the purified meso-lactide stream based on the combined weight of the purified meso-lactide stream and the purified S,S- and R,R-lactide stream, and polymerizing the recombined stream.

**2.** The process of claim 1, wherein a portion of the purified meso-lactide stream is recycled to steps a) and/or b) of the process.

**3.** The process of claim 1 or 2, wherein step c) is performed by distillation and the meso-lactide-enriched stream is a gas stream.

**4.** The process of claim 3, wherein step d) is performed in a melt crystallizer and the meso-lactide-enriched stream is condensed prior to entering the melt crystallizer.

**5.** The process of any preceding claims, wherein step d) is performed in one or more of a static melt crystallizer, falling film melt crystallizer, suspension crystallizer or suspension mixed product removal crystallizer.

**6.** The process of any preceding claims, wherein crystals of meso-lactide form in step d).

**7.** The process of claim 6, wherein the crystals of meso-lactide are further purified by one or more of washing, heating them close to their melting temperature to exclude entrapped impurities and recrystallization.

**8.** The process of claim 6, wherein the purified meso-lactide stream recombined with the purified S,S- and R,R-lactide stream in step e) contains i) up to 250 ppm acetic acid; ii) up to 400 ppm succinic acid, iii) up to 75 ppm pyruvic acid, iv) up to 125 ppm levulinic acid, v) up to 1000 ppm 2,3-butanediol and/or vi) up to 750 ppm 2-methyl succinic acid.

## Patentansprüche

**1.** Verfahren zum Herstellen eines Polylactids, umfassend:

a) Bilden einer Poly(milchsäure) mit niedrigem Molekulargewicht,
b) Depolymerisieren der Poly(milchsäure) mit niedrigem Molekulargewicht, um ein Rohlactid zu bilden, das Meso-Lactid, S,S-Lactid und R,R-Lactid beinhaltet, wobei entweder S,S-Lactid oder R,R-Lactid das nicht vorherrschende Lactid ist, und das Rohlactid ferner mittelsiedende Unreinheiten beinhaltet,
c) Trennen von Meso-Lactid von dem Rohlactid in einem oder mehreren Schritten, sodass

1) ein mit Meso-Lactid angereicherter Strom gebildet wird, in dem die Molfraktion von Meso-Lactid zumindest 0,8 ist und in dem die mittelsiedenden Unreinheiten konzentriert sind, und
2) ein gereinigter S,S- und R-R-Lactid-Strom gebildet wird,

d) Reinigen des mit Meso-Lactid angereicherten Stroms durch Schmelzkristallisierung, um Meso-Lactid von mittelsiedenden Unreinheiten zu trennen und einen gereinigten Meso-Lactid-Strom zu bilden,
e) Rekombinieren von zumindest einem Teil des gereinigten Meso-Lactid-Stroms mit zumindest einem Teil des gereinigten S,S- und R-R-Lactid-Stroms, um einen rekombinierten Strom zu bilden, der 4 bis 25 Gewichts-% des gereinigten Meso-Lactid-Stroms basierend auf dem kombinierten Gewicht aus dem gereinigten Meso-Lactid-Strom und dem gereinigten S,S- und R-R-Lactid-Strom enthält, und Polymerisieren des rekombinierten Stroms.

**2.** Verfahren nach Anspruch 1, wobei ein Teil des gereinigten Meso-Lactid-Stroms zu Schritt a) und/oder b) des Verfahrens zurückgeführt wird.

**3.** Verfahren nach Anspruch 1 oder 2, wobei Schritt c) durch Destillation durchgeführt wird und der mit Meso-Lactid angereicherte Strom ein Gasstrom ist.

**4.** Verfahren nach Anspruch 3, wobei Schritt d) in einem Schmelzkristallisator durchgeführt wird und der mit Meso-Lactid angereicherte Strom kondensiert wird, bevor er in den Schmelzkristallisator eintritt.

**5.** Verfahren nach einem der vorhergehenden Ansprüche, wobei Schritt d) in einem oder mehreren von einem statischen Schmelzkristallisator, einem Rieselfilmschmelzkristallisator, einem Suspensionskristallisator oder einem Suspensionsmischprodukt-Entfernungskristallisator durchgeführt wird.

**6.** Verfahren nach einem der vorhergehenden Ansprüche, wobei Kristalle aus Meso-Lactid in Schritt d) gebildet werden.

**7.** Verfahren nach Anspruch 6, wobei die Kristalle aus Meso-Lactid durch eines oder mehrere von Waschen, Erwärmen auf nahe ihrer Schmelztemperatur, um eingeschlossene Unreinheiten auszuschließen, und Rekristallisierung weiter gereinigt werden.

**8.** Verfahren nach Anspruch 6, wobei der gereinigte Meso-Lactid-Strom, der in Schritt e) mit dem gereinigten S,S- und R,R-Lactid-Strom rekombiniert wird, i) bis zu 250 ppm Essigsäure, ii) bis zu 400 ppm Bernsteinsäure, iii) bis zu 75 ppm Brenztraubensäure, iv) bis zu 125 ppm Lävulinsäure, v) bis zu 1000 ppm 2,3-Butandiol und/oder vi) bis zu 750 ppm 2-Methyl-Bernsteinsäure enthält.

## Revendications

**1.** Procédé de production d'un polylactide, comprenant :

a) la formation d'un acide polylactique de faible masse moléculaire ;
b) la dépolymérisation de l'acide polylactique de faible masse moléculaire pour former un lactide brut qui inclut du méso-lactide, du S,S-lactide et du R,R-lactide, où soit le S,S-lactide soit le R,R-lactide est le lactide non-prédominant, et le lactide brut inclut en outre des impuretés à point d'ébullition intermédiaire ;
c) la séparation du méso-lactide du lactide brut en une ou plusieurs étapes de sorte que

1) un courant enrichi en méso-lactide est formé dans lequel la fraction molaire du méso-lactide est d'au moins 0,8 et dans lequel les impuretés à point d'ébullition intermédiaire sont concentrées ; et
2) un courant de S,S- et de R,R-lactide purifié est formé ;

d) la purification du flux enrichi en méso-lactide par cristallisation à l'état fondu pour séparer le méso-lactide des impuretés à point d'ébullition intermédiaire et former un courant de méso-lactide purifié ;
e) la recombinaison d'au moins une partie du courant de méso-lactide purifié avec au moins une partie du courant de S,S- et de R,R-lactide purifié pour former un courant recombiné contenant 4 à 25 % en masse du courant de méso-lactide purifié par rapport à la masse combinée du courant de méso-lactide purifié et du courant de S,S- et de R,R-lactide purifié, et la polymérisation du courant recombiné.

2. Procédé selon la revendication 1, dans lequel une partie du courant de méso-lactide purifié est recyclé aux étapes a) et/ou b) du procédé.

3. Procédé selon la revendication 1 ou 2, dans lequel l'étape c) est réalisée par distillation et le courant enrichi en méso-lactide est un courant gazeux.

4. Procédé selon la revendication 3, dans lequel l'étape d) est réalisée dans un cristallisoir de fonte et le courant enrichi en méso-lactide est condensé avant d'entrer dans le cristallisoir de fonte.

5. Procédé selon l'une des revendications précédentes, dans lequel l'étape d) est réalisée dans un ou plusieurs des éléments suivants : un cristallisoir de fonte statique, un cristallisoir de fonte à film tombant, un cristallisoir à suspension, et un cristallisoir d'élimination des produits mixtes en suspension.

6. Procédé selon l'une des revendications précédentes, dans lequel des cristaux de méso-lactide se forment à l'étape d).

7. Procédé selon la revendication 6, dans lequel les cristaux de méso-lactide sont en outre purifiés par un ou plusieurs des procédés suivants : lavage, chauffage à une température proche de leur température de fusion pour exclure les impuretés piégées, et recristallisation.

8. Procédé selon la revendication 6, dans lequel le courant de méso-lactide purifié recombiné avec le courant de S,S- et de R,R-lactide purifié dans l'étape e) contient i) jusqu'à 250 ppm d'acide acétique, ii) jusqu'à 400 ppm d'acide succinique, iii) jusqu'à 75 ppm d'acide pyruvique, iv) jusqu'à 125 ppm d'acide lévulinique, v) jusqu'à 1000 ppm de 2,3-butanediol et/ou vi) jusqu'à 750 ppm d'acide 2-méthyl succinique.

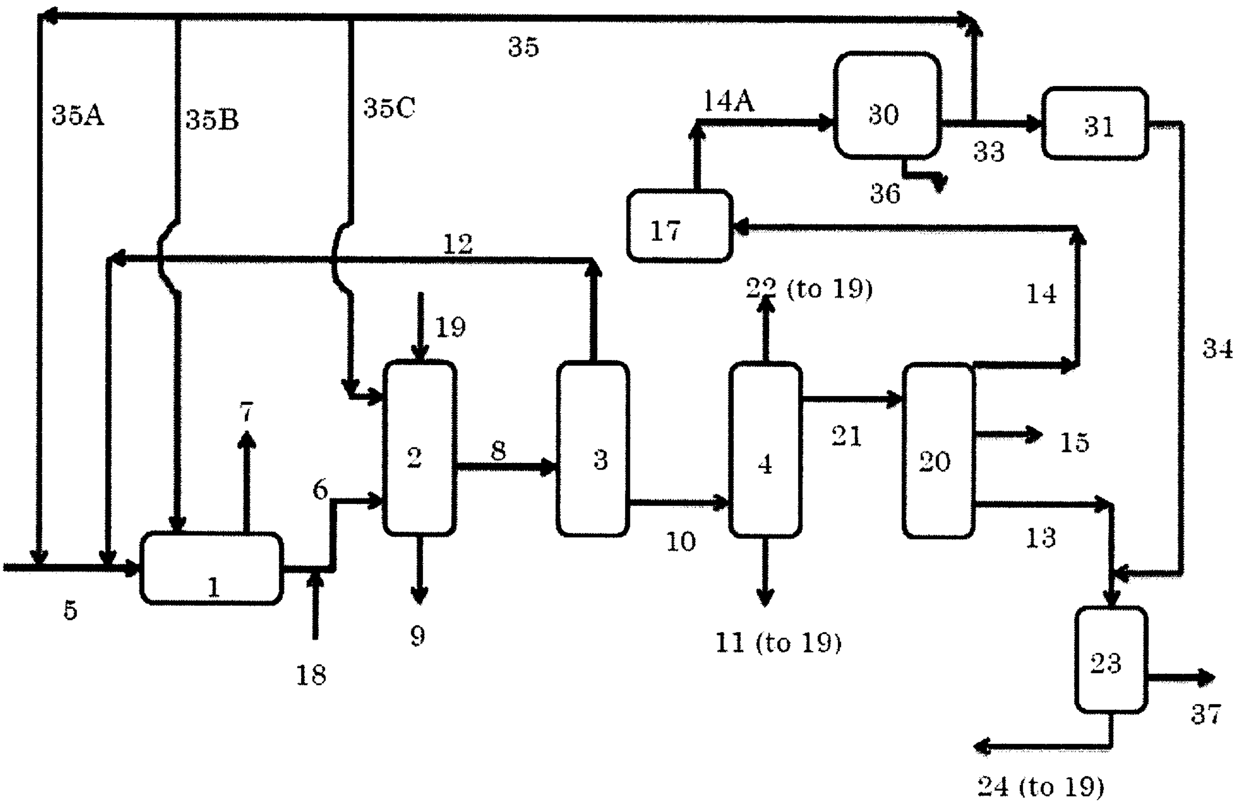
35
35A
35B
35C
14A
30
31
33
36
12
17
22 (to 19)
14
19
34
7
2
8
3
4
21
20
15
6
10
13
1
5
9
11 (to 19)
18
23
37
24 (to 19)

Fig.1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5247058 A
- US 5258488 A
- US 5357035 A
- US 5338822 A
- US 6005067 A
- US 6277951 B
- US 6326458 B
- WO 9315127 A
- US 20140031566 A
- WO 2014180836 A
- WO 2010105143 A
- WO 9509879 A
- US 6310218 B
- US 5247059 A
- US 5274073 A
- US 5208667 A, McLain